# EUROPEAN PATENT SPECIFICATION

(11) **EP 2 996 587 B1**
(45) Date of publication and mention of the grant of the patent: **24.08.2022**
(21) Application number: 14716957.7
(22) Date of filing: 25.03.2014
(51) Int. Cl.: A61B 17/34, A61N 5/10, A61B 17/00, A61B 34/20, A61B 18/00, A61B 90/00, A61B 8/08, A61B 8/12

(54) **INSTRUMENT LOCALIZATION IN GUIDED HIGH DOSE RATE BRACHYTHERAPY**
INSTRUMENTENLOKALISIERUNG IN EINER GEFÜHRTEN BRACHYTHERAPIE MIT HOHER DOSIERUNGSRATE
LOCALISATION D'INSTRUMENT EN CURIETHÉRAPIE A HAUT DÉBIT DE DOSE

(30) Priority: 28.03.2013 US 201361805965 P
(43) Date of publication of application: 23.03.2016
(73) Proprietor: Koninklijke Philips N.V., 5656 AG Eindhoven (NL)
(72) Inventor: DEHGHAN MARVAST, Ehsan, 5656 AE Eindhoven (NL)
(74) Representative: Philips Intellectual Property & Standards
(86) International application number: PCT/IB2014/060130
(87) International publication number: WO 2014/155285

(56) References cited:
- EP-A1- 1 932 477
- WO-A1-99/33394
- WO-A1-2006/089426
- US-A1- 2001 041 838
- US-A1- 2012 016 316

## Description

### BACKGROUND:

### Technical Field

This disclosure relates to medical instruments and more particularly to systems and methods for high dose rate (HDR) brachytherapy using image based device registration.

### Description of the Related Art

High Dose Rate (HDR) brachytherapy is a treatment for prostate cancer in which some radioactive sources are temporarily introduced into the prostate gland through several hollow catheters to kill the cancerous tissue. In ultrasound (US) guided HDR brachytherapy, the hollow catheters are implanted inside the prostate, based on a plan, by passing the catheters through a guiding grid. Then, a three-dimensional (3D) US volume is generated by translating a Transrectal Ultrasound (TRUS) probe from base to apex. The prostate and the catheters are segmented on the images, and the position of the catheters is sent to a computer, which optimizes a location of the radioactive sources inside the catheters (dwell positions) and the amount of time (dwell time) that the radioactive source should be present at the dwell positions. A plan is then executed using an after loader. A drawback of this approach is that catheter segmentation in US images is cumbersome and difficult due to shadowing and calcifications.

Electromagnetic (EM) trackers have been proposed to localize the catheters. In this type of system, a probe is tracked with an EM-tracker to create a 3D ultrasound volume by retraction of the probe from prostate base to apex. Also, the guiding grid is related to the ultrasound volume using an EM tracked pointer in a calibration phase. The relationship between EM trackers and the ultrasound volume is known after the calibration phase. After insertion of the catheters, an EM-tracked guide-wire is inserted through the catheters to localize them in the ultrasound volume.

Even after careful calibration, there is some error between the catheter's locations identified using EM-tracking and the real position of the catheters that appears as bright regions in the ultrasound volume. If not corrected, this error can result in significant under- or over-dosage of the tissue. Reasons for this error can be a result of an original calibration error between the US image and the probe EM tracker and also variations in the magnetic field of the EM field-generator caused by metallic objects nearby, or simply by changes in the readings of the EM trackers caused by different distances and/or orientation of a field-generator. Attempts to reduce this error include manually segmenting some catheters in ultrasound and registering them to EM-tracked positions. Of course, manual segmentation of the catheters is time consuming, cumbersome and subject to human error. International Patent Application WO2006/089426 and European Patent EP1932477 disclose medical systems that include position detection.

### SUMMARY

The invention is defined in the appended claims.

The objects, features and advantages of the present disclosure will become apparent from the following detailed description of illustrative embodiments thereof, which is to be read in connection with the accompanying drawings.

### BRIEF DESCRIPTION OF DRAWINGS

This disclosure will present in detail the following description of preferred embodiments with reference to the following figures wherein:
FIG. 1 is a block/flow diagram showing a system for instrument localization using ultrasound images in accordance with one embodiment;
FIG. 2 is a schematic perspective view of the system of FIG. 1 in accordance with one embodiment;
FIG. 3 is a flow diagram showing a method for visual instrument localization in accordance with an illustrative embodiment;
FIG. 4 is a flow diagram showing another method for visual instrument localization in accordance with another illustrative embodiment; and
FIG. 5 is a diagram depicting implantation of catheters for brachytherapy using a guidance grid in accordance with one illustrative embodiment.

### DETAILED DESCRIPTION OF EMBODIMENTS

In accordance with the present principles, systems and methods for automatic localization of medical instruments, such as catheters or needles, in ultrasound (US) are provided. An image based registration method is employed to register positions of the catheters in an electromagnetic (EM) field-generator coordinate system to an ultrasound coordinate system. This method obviates the need for EM-grid registration and also corrects for conventional errors by registering the EM-tracked catheters directly to the US coordinate system, using the US image content. The present methods remove the restriction of using an EM-tracked probe, and may instead employ an optically tracked 2D US probe or a non-tracked 3D probe. In addition, the present embodiments may be employed to register an EM field-generator coordinate system to an ultrasound coordinate system without using an EM-tracked 2D ultrasound probe. In this case, a conventional system including an optically tracked ultrasound probe can be used to simplify the application.

It should be understood that the present invention will be described in terms of medical instruments; however, the teachings of the present invention are much broader and are applicable to any ultrasonic tracking system. In some embodiments, the present principles are employed in tracking or analyzing complex biological or mechanical systems. In particular, the present principles are applicable to internal tracking procedures of biological systems, procedures in all areas of the body such as the lungs, gastro-intestinal tract, excretory organs, blood vessels, prostate, kidneys, etc. The elements depicted in the FIGS. may be implemented in various combinations of hardware and software and provide functions which may be combined in a single element or multiple elements.

The functions of the various elements shown in the FIGS. can be provided through the use of dedicated hardware as well as hardware capable of executing software in association with appropriate software. When provided by a processor, the functions can be provided by a single dedicated processor, by a single shared processor, or by a plurality of individual processors, some of which can be shared. Moreover, explicit use of the term "processor" or "controller" should not be construed to refer exclusively to hardware capable of executing software, and can implicitly include, without limitation, digital signal processor ("DSP") hardware, read-only memory ("ROM") for storing software, random access memory ("RAM"), non-volatile storage, etc.

Furthermore, embodiments of the present invention can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium. Examples of a computer-readable medium include a semiconductor or solid state memory, magnetic tape, a removable computer diskette, a random access memory (RAM), a read-only memory (ROM), a rigid magnetic disk and an optical disk. Current examples of optical disks include compact disk - read only memory (CD-ROM), compact disk - read/write (CD-R/W), Blu-Ray^{™} and DVD

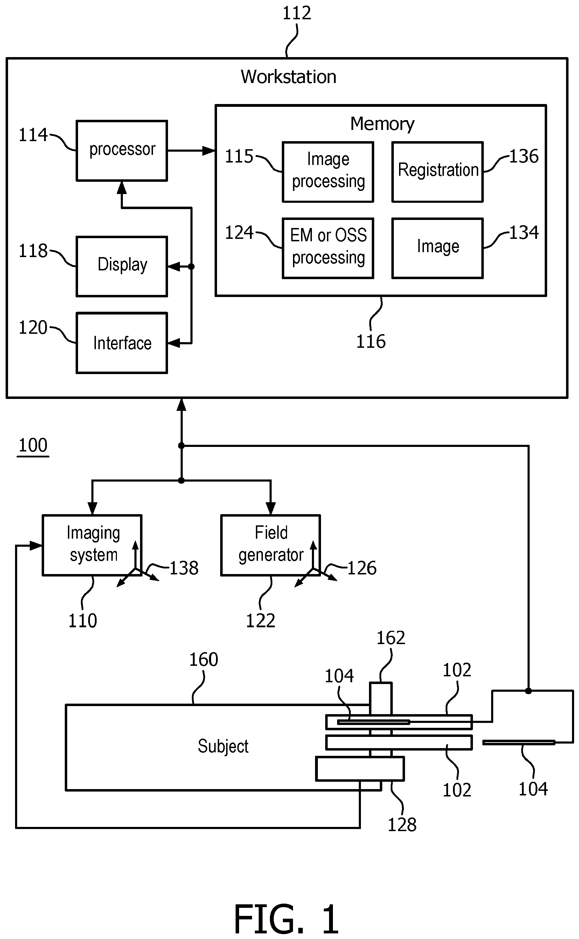

Referring now to the drawings in which like numerals represent the same or similar elements and initially to FIG. 1, a system 100 for guiding one or more medical instruments using localization by ultrasonic images is illustratively shown in accordance with one embodiment. System 100 may include a workstation or console 112 from which a procedure is supervised and/or managed. Workstation 112 preferably includes one or more processors 114 and memory 116 for storing programs and applications. Memory 116 may store an image processing module 115 configured to interpret and compare images from an ultrasonic imaging system 110. Image processing module 115 is configured to collect images using an ultrasound (US) probe 128. The US probe 128 may include a transrectal US (TRUS) probe as the present principles will be illustratively described in terms of a high-dose-rate (HDR) brachytherapy procedure. The probe 128 may employ an optically tracked US probe, an EM-tracked US probe or a non-tracked 3D probe.

During such a procedure, one or more medical instruments 102, such as one or more catheters and/or other instruments are inserted into a subject 160 (e.g., a patient). In a brachytherapy application, the one or more instruments 102 are passed through a guidance grid 162. The guidance grid 162 is calibrated/registered with a coordinate system 138 of the imaging system 110 in advance of a procedure (i.e., *a priori*)*.* The one or more instruments 102 are configured to include or receive tracking devices 104 therein, e.g., EM tracking and/or optical shape sensing devices/systems. For example, in HDR brachytherapy, the one or more catheters 102 do not have tracking devices 104 embedded in them. The one or more catheters 102, which are hollow tubes, are inserted into the tissue (subject 160). Then, a shape reconstructing device and/or tracking device 104, such as an EM-tracked guidewire and/or an optical shape sensing fiber is inserted into the one or more catheters 102 and removed. In fact, one EM-tracked guidewire or optical shape sensing (OSS) fiber can be used to reconstruct the shape of several catheters. In other embodiments, the one or more catheters 102 and the one or more shape-reconstructing sensors and/or tracking devices 104 may also be integrated together.

For EM tracking, positions of the one or more instruments 102 are tracked using a field generator 122 and a tracking system processing module 124 (e.g., for EM tracking). The EM field is generated, and the movements of the instruments 102 are tracked in an EM coordinate system 126. For OSS fiber tracking, positions of the one or more instruments 102 are tracked using feedback from a fiber optic device the tracking system processing module 124 (e.g., for optical shape sensing). The back reflected light generated by the shapes of the fiber device are tracked in their own coordinate system (e.g., the coordinate system 126). While only a single tracking method is needed, both systems may be employed together, or other tracking systems may be employed.

In accordance with one embodiment, the one or more instruments 102 appear as one or more bright regions in a US volume rendered on a display 118. An image-based approach can be used to overlay or register the EM-tracked catheter positions from the EM processing module 124 onto the US volume in the image processing module 115 using a registration module 136, and, hence, localize the one or more catheters 102 in the US volume. In other embodiments, the EM processing may be replaced with optical shape sensing in module 124, and the EM tracking devices 104 may be replaced by optical fibers for shape sensing. EM signals or optical signals are employed to determine the positions of the one or more instruments 102. The one or more instruments 102 preferably include one or more catheters but may include a guidewire, a probe, an endoscope, other medical component, etc.

In one embodiment, workstation 112 includes the display 118 for viewing internal images of the subject (patient) 160 and may include an image 134 of the volume as an overlay of another image or rendering. Display 118 may also permit a user to interact with the workstation 112 and its components and functions, or any other element within the system 100. This is further facilitated by an interface 120 which may include a keyboard, mouse, a joystick, a haptic device, or any other peripheral or control to permit user feedback from and interaction with the workstation 112.

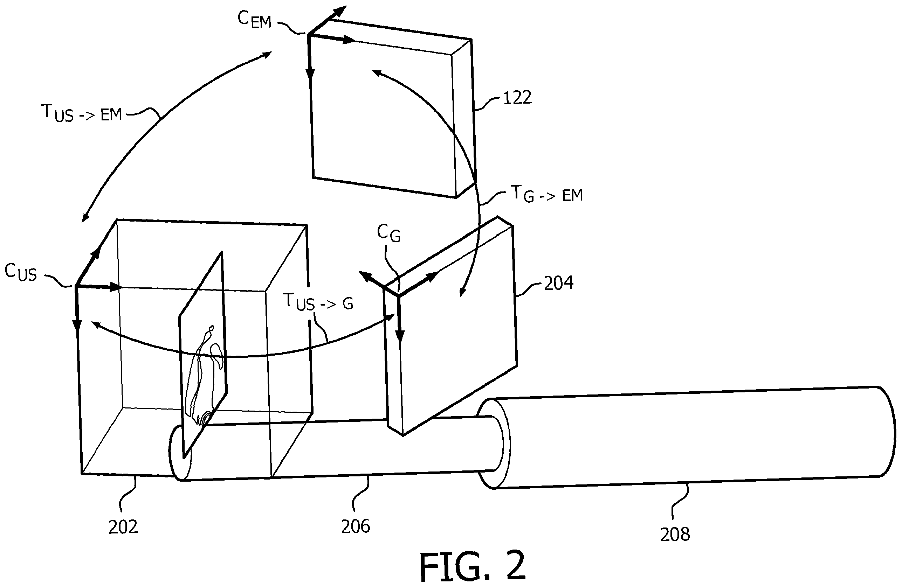

Referring to FIG. 2, a schematic diagram shows a system setup which employs EM-tracking and US. Let C_{US} be a coordinate system of a US volume 202, while C_{EM} is a coordinate system of an EM field-generator 122 and C_{G} is the coordinate system of a grid 204. The grid 204 is a guiding or guidance grid employed to assist in the delivery and positioning of instruments 102 (e.g., catheters, needles, etc.). A transformation T_{US→EM}, transforms the coordinate system from C_{US} to C_{EM}, and T_{G→EM} transforms the coordinate system from C_{G} to C_{EM}.

In one embodiment, a TRUS probe 206 is equipped with EM trackers, therefore T_{US→EM} and T_{G→EM} can be identified during a calibration process. This calibration may not be sufficiently accurate for brachytherapy purposes. Nonetheless, it may be used as an initial position for the registration process.

In another embodiment, the TRUS probe 206 is not tracked with an EM tracker. Alternatively, an optical encoder or any other encoder 208 can be used to track the probe 206. In this case, T_{US→EM} is not known and needs to be identified in accordance with the present principles. In yet another embodiment, optical shape sensing may be employed to detect the shape of the catheters (102). In this case, a T_{US→Op} should be identified instead of the T_{US→EM}. In still another embodiment, a 3D probe is employed for the application which is not necessarily tracked.

In all of these embodiments, T_{US→G} can be identified using a needle in a water tank (this is a clinical method). This transformation is rather stable and does not need to be repeated each time.

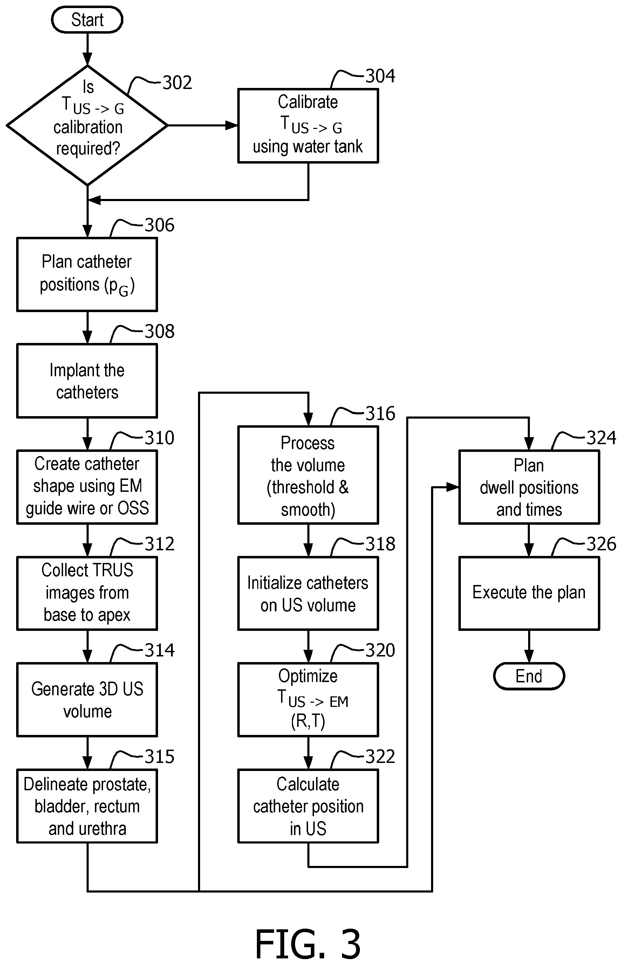

Referring to FIG. 3 with continued reference to FIG. 2, a method for image based tracking of medical instruments using US is shown in accordance with illustrative embodiments. In block 302, whether calibration of T_{US→G} is needed is determined. If yes, T_{US→G} is calibrated in block 304. In no, block 306 is visited. The calibration may include using a needle in a water tank or other calibration method.

In block 306, a plan is developed for the placement of catheters or other medical instruments (p_{G}). In block 308, the catheters or other medical instruments are implanted inside tissue based on the plan. The planned catheter positions are in a grid coordinate system C_{G}. The planned position p_{G} can be transformed to C_{US} by: pus = (T_{US→G})⁻¹. p_{G}.

In block 310, position sensors, such as, e.g., an EM guide-wire or and an optical shape sensor (OSS), are inserted into the instruments (e.g., catheters) to create the shape of the catheters in the EM field-generator coordinate system (C_{EM}) or the OSS coordinate system (C_{OSS}). We will assume the catheter positions are c_{TR}. In block 312, ultrasound images are collected to create an ultrasound volume in block 314. This may be generated by retraction of a tracked TRUS probe from base to apex or using a 3D US probe. In block 315, organs or regions of interest within the US volume are delineated for image viewing. This may be performed using image processing 115 (FIG. 1). The delineation or organs may be employed in planning, in block 324, and may be employed in visualization of the catheters proceeding with block 316.

In accordance with the present principles, the one or more instruments can be visually tracked within the body. By providing delineated organs in a same image, a user can decipher exactly where the one or more instruments and hence treatment agents will be or are delivered. This can be employed to more accurately carry out a plan and to make corrections in real-time for any errors that may be encountered. In addition, real-time visual feedback is enabled, which can increase user confidence that the appropriate treatment is being performed in accordance with the plan. With real-time visualization of an organ of interest, say the prostate, the user can immediately evaluate the instrument positions to ensure they are placed within the boundaries of the organ and there is less likelihood of over or under treatment.

In block 316, the US volume is processed by the image processing module (115, FIG. 1) to threshold the volume to keep the bright regions only and/or to use a Gaussian filter to smooth the image. It should be understood that different filters or image processing techniques may be employed to enhance image viewing and discovery of image features or artifacts to improve accuracy. In block 318, the tracked catheter points are initialized in the C_{US} coordinate system by rigidly registering them to p_{US}. We assume the registered catheter positions are c_{US}⁰, so that c_{US}⁰ = R⁰.c_{TR}+T⁰. In block 320, the overlap of c_{US}⁰ and the processed US volume are quantified by summing the intensity of US volume at c_{US}⁰ and iteratively, optimizing T_{US→EM} (optimizing (Rⁱ, T')) to maximize the overlap. In other words, the rotation (R) and translation (T) between tracked data and image data are optimized to find a best fit therebetween.

In block 322, catheter positions are computed in the US coordinate system. This computation is done in real-time to permit the visual tracking of the catheter position in the US images in a display. In this way, a user can visually track the catheters to ensure compliance with the plan. The positions of the catheters are recomputed and updated visually as needed, and preferably in real-time or near-real-time. In block 324, dwell positions and times may be computed using the catheter positions and delineations of organs of interest both in the ultrasound coordinate system as part of the plan or at any time during the procedure to update the plan. The dose and amount of dwell time may be computed for high dose rate (HDR) brachytherapy or any other procedure. The organs of interest should be delineated in the US volume for dose planning in block 315. One benefit of the EM-US or OSS-US registration method is that after the registration, both catheters and the organ contours are in the same coordinate system and can be used for tracking. In block 326, the plan is executed or modified and executed to achieve the goals of the procedure.

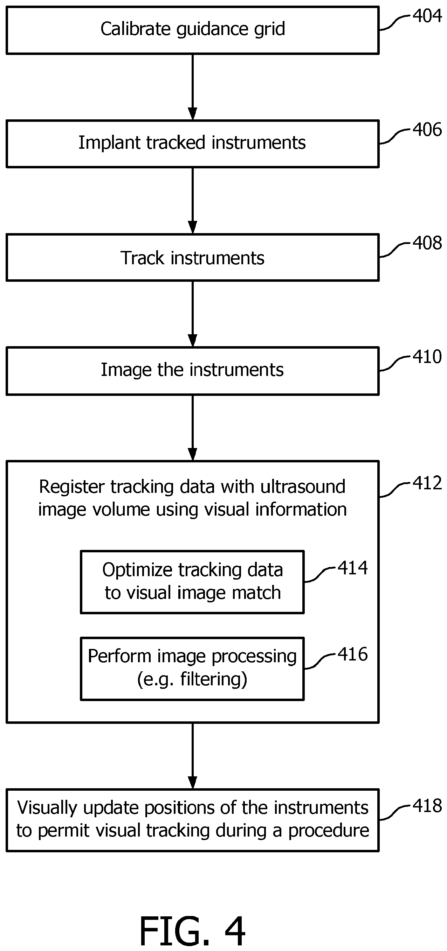

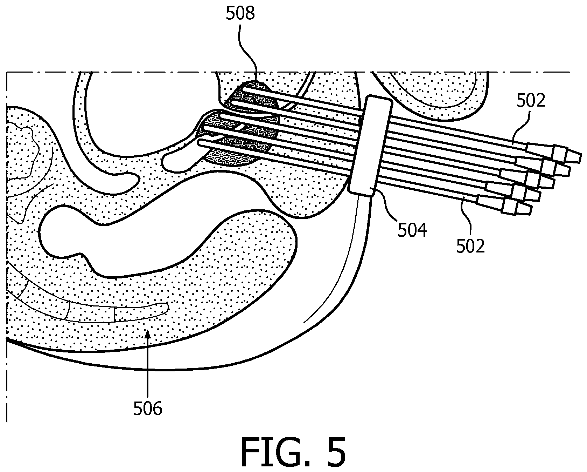

Referring to FIG. 4, a method for visually localizing one or more medical instruments is illustratively depicted. In block 404, a guidance grid is calibrated to an imaging system for ultrasonically imaging one or more medical instruments. In block 406, the one or more medical instruments are implanted in a subject using the guidance grid. This is illustratively depicted in FIG. 5 where the medical instruments include one or more catheters 502 and are passed through a guidance grid or template 504 into a subject 506. In this case, the implantation is for treatment of a prostate 508; however, other procedures are contemplated. In block 408, positions of the one or more medical instruments are tracked using a tracking system. The tracking system may include an electromagnetic tracking system, an optical shape sensing system and/or other tracking device or system.

In block 410, the one or more medical instruments are ultrasonically imaged in an image volume where the one or more medical instruments are deployed. The imaging system may include an ultrasonic probe that is not tracked to permit flexibility in placement of the probe. The probe may include a transrectal probe ultrasonic (TRUS) probe.

In block 412, tracked positions of the one or more medical instruments are registered to visual positions of the one or more medical instruments. The one or more medical instruments preferably include one or more catheters, which appear in the volume as one or more bright regions. In block 414, registration is optimized by finding a best fit between the bright regions and tracked positions of the one or more medical instruments. This may include minimizing rotation and translation between images and tracking data. In block 416, the image volume may be further processed to threshold and/or filter to highlight the bright regions and to highlight organ boundaries for improved visualization of the procedure. In block 418, position changes determined by the tracking system are updated visually in the image volume during a procedure. The one or more medical instruments are employed to deliver treatment during, e.g., a brachytherapy procedure.

## Claims

1. A system (100) for localizing one or more medical instruments (102), comprising:
a tracking system (104) configured to track the positions of the one or more medical instruments (102), thereby providing tracked position information (c_{TR}) of the one or more medical instruments;
a guidance grid (162) configured to be disposed in an operative relationship with a subject (160) and configured to receive the one or more medical instruments (102), such that planned positions (p_{G}) of the one or more medical instruments are defined with respect to the guidance grid;
an ultrasound imaging system (110) configured to image a volume (202) where the one or more medical instruments are deployed, thereby providing visual positions (c_{US}) of the one or more medical instruments, wherein the guidance grid (162) is calibrated, a priori, to the imaging system, such that the planned positions (p_{G}) of the one or more medical instruments with respect to the guidance grid are transformable to planned positions (p_{US}) of the one or more medical instruments with respect to the volume;
a display (118); and
a program module (136) configured to register the tracked position information (c_{TR}) of the one or more medical instruments to the visual positions of the one or more medical instruments in the volume, using the planned positions (p_{US}) of the one or more medical instruments with respect to the volume, for display on the display (118) such that the tracked positions of the one or more medical instruments are overlaid on image data of the volume to permit a user to visually track the one or more medical instruments on the display of the imaging system.

2. The system as recited in claim 1, wherein the tracking system (104) includes one of an electromagnetic tracking system and an optical shape sensing system (104).

3. The system as recited in claim 1, wherein the ultrasound imaging system includes a probe (128) that is not tracked.

4. The system as recited in claim 3, wherein the probe (128) includes a transrectal probe ultrasonic (TRUS) probe.

5. The system as recited in claim 1, wherein the one or more medical instruments (102) include catheters, which appear in the volume as bright regions such that the bright regions are employed to register the positions determined by the tracking system.

6. The system as recited in claim 1, further comprising an image processing module (115) configured to at least one of threshold or filter the image to highlight the bright regions.

7. The system as recited in claim 1, wherein the one or more medical instruments (102) are employed to deliver treatment during a brachytherapy procedure.

8. The system as recited in claim 1, wherein one or more regions of interest are delineated in an image along with the tracked positions of the one or more medical instruments.

## Patentansprüche

1. Ein System (100) für die Ortsbestimmung eines oder mehrerer medizinischer Instrumente (102),
umfassend:
Ein Nachverfolgungssystem (104), mit dem man die Position des einen oder der mehreren medizinischen Instrumente (102) nachverfolgt und auf diese Weise die nachverfolgten Positionsinformationen (c_{TR}) des einen oder der mehreren medizinischen Instrumente bereitstellt;
Ein Führungsraster (162), konfiguriert, um in einer operativen Umgebung bei einem Patienten (160) eingesetzt zu werden, und konfiguriert, um aufzunehmen:
Das eine oder die mehreren medizinischen Instrumente (102), sodass die geplanten Positionen (p_{G}) des einen oder der mehreren medizinischen Instrumente in Bezug zum Führungsraster definiert sind;
ein Ultraschall-Bildgebungssystem (110), konfiguriert, um ein Volumen (202) abzubilden, wo das eine oder die mehreren medizinischen Instrumente eingesetzt sind, dadurch visuelle Positionen (c_{US}) des einen oder der mehreren medizinischen Instrumente bereitstellend, wobei das Führungsraster (162) kalibriert ist, zunächst einmal, gegen das Bildgebungssystem, sodass die geplanten Positionen (p_{G}) des einen oder der mehreren medizinischen Instrumente in Bezug auf das Führungsraster sich umwandeln lassen zu den geplanten Positionen (p_{US}) des einen oder der mehreren medizinischen Instrumente in Bezug zum Volumen;
ein Display (118); und ein Programm-Modul (136), konfiguriert, um die nachvollzogene Positionsinformation (c_{TR}) des einen oder der mehreren medizinischen Instrumente an den optischen Positionen des einen oder der mehreren medizinischen Instrumente im Volumen zu registrieren, unter Verwendung der geplanten Positionen (p_{US}) des einen oder der mehreren medizinischen Instrumente in Bezug zum Volumen, für eine Anzeige auf dem Display (118), sodass die nachverfolgten eines oder mehrere medizinische Instrumente den Bilddaten des Volumens überlagert sind, damit die Benutzer optisch den Weg des einen oder der mehreren medizinischen Instrumente auf dem Display des Bildgebungssystems nachvollziehen können.

2. System wie in Anspruch 1 angegeben, wobei das System zum Nachverfolgen (104) entweder ein elektromagnetisches System zur Nachverfolgung oder ein optisches System zum Fühlen von Formen (104) enthält.

3. System wie in Anspruch 1 angegeben, wobei das Ultraschall-Bildgebungssystem eine Sonde (128) enthält, die nicht nachverfolgt wird.

4. System wie in Anspruch 3 angegeben, wobei die Sonde (128) eine transrektale Ultraschallsonde (TRUS) enthält.

5. System wie in Anspruch 1 angegeben, wobei das eine oder die mehreren medizinischen Instrumente (102) Katheter umfassen, die in dem Volumen als helle Bereiche erscheinen, sodass die hellen Bereiche verwendet werden, um die durch das Verfolgungssystem bestimmten Positionen zu registrieren.

6. System wie in Anspruch 1 angegeben, welches ferner ein Bildverarbeitungsmodul (115) umfasst, das so konfiguriert ist, dass es mindestens einen Schwellwert für das Bild festlegt oder es filtert, um die hellen Bereiche hervorzuheben.

7. System wie in Anspruch 1 angegeben, wobei das eine oder die mehreren medizinischen Instrumente (102) verwendet werden, um während eines Brachytherapieverfahrens eine Behandlung bereitzustellen.

8. System wie in Anspruch 1 angegeben, wobei der eine oder die mehreren interessierenden Bereiche in einem Bild entlang der nachverfolgten Position des einen oder der mehreren medizinischen Instrumente markiert werden.

## Revendications

1. Système (100) pour localiser un ou plusieurs instruments médicaux (102),
comprenant:
un système de suivi (104) configuré pour suivre les positions de l'un ou de plusieurs instruments médicaux (102), fournissant ainsi des informations de position suivie (c_{TR}) de l'un ou de plusieurs instruments médicaux;
une grille de guidage (162) configurée pour être disposée dans une relation opérationnelle avec un sujet (160) et configurée pour recevoir
un ou plusieurs instruments médicaux (102), de telle sorte que les positions planifiées (p_{G}) du ou des instruments médicaux sont définies par rapport à la grille de guidage;
un système d'imagerie à ultrasons (110) configuré pour afficher un volume (202)
où le ou les
plusieurs instruments médicaux sont déployés, fournissant ainsi des positions visuelles (c_{US}) du ou des instruments médicaux, dans lequel la grille de guidage (162) est calibrée, a priori, au système d'imagerie, de sorte que les positions planifiées (p_{G}) du ou des instruments médicaux par rapport à la grille de guidage sont transformables en positions planifiées (p_{US}) du ou des instruments médicaux par rapport au volume;
un écran (118); et
un module de programme (136) configuré pour enregistrer les informations de position suivie (c_{TR}) des un ou plusieurs instruments médicaux par rapport au volume, en utilisant les positions planifiées (p_{US}) du un ou plusieurs instruments médicaux par rapport au volume, pour un affichage sur l'écran (118) de telle sorte que les positions suivies des un ou plusieurs instruments médicaux sont superposées aux données d'image du volume pour permettre à l'utilisateur de suivre visuellement les un ou plusieurs instruments médicaux sur l'écran du système d'imagerie.

2. Système selon la revendication 1, dans lequel le système de suivi (104) comprend l'un d'un système de suivi électromagnétique et d'un système de détection de forme optique (104).

3. Système tel que décrit dans la revendication 1, dans lequel le système d'imagerie à ultrasons comprend une sonde (128) qui n'est pas suivie.

4. Système tel que décrit dans la revendication 3, dans lequel la sonde (128) comprend une sonde transrectale à ultrasons (TRUS).

5. Système tel que décrit dans la revendication 1, dans lequel le ou les instruments médicaux (102) comprennent des cathéters, qui apparaissent dans le volume comme des régions lumineuses de sorte que les régions lumineuses sont utilisées pour enregistrer les positions déterminées par le système de suivi.

6. Système tel que décrit dans la revendication 1, comprenant en outre un module de traitement d'image (115) configuré pour au moins l'un d'un seuil ou d'un filtre de l'image pour mettre en évidence les régions lumineuses.

7. Système tel que décrit dans la revendication 1, dans lequel le ou les instruments médicaux (102) sont utilisés pour délivrer un traitement pendant une procédure de curiethérapie.

8. Système selon la revendication 1, dans lequel une ou plusieurs régions d'intérêt sont délimitées dans une image avec les positions suivies d'un ou plusieurs instruments médicaux.
